# EUROPEAN PATENT SPECIFICATION

(11) **EP 2 659 258 B1**
(45) Date of publication and mention of the grant of the patent: **09.12.2015**
(21) Application number: 11804619.2
(22) Date of filing: 13.12.2011
(51) Int. Cl.: B07C 5/344, C12Q 1/04, G01N 21/3563, G01N 21/3577, G01N 21/3581, G01N 21/90, G01N 21/94

(54) **HIGH SPEED SPOILAGE DETECTION USING AN ELECTROMAGNETIC SIGNAL**
HOCHGESCHWINDIGKEITSNACHWEIS VON VERDERB ANHAND EINES ELEKTROMAGNETISCHEN SIGNALS
DÉTECTION À GRANDE VITESSE D'UNE DÉTÉRIORATION À L'AIDE D'UN SIGNAL ÉLECTROMAGNÉTIQUE

(30) Priority: 30.12.2010 US 201061428647 P
(43) Date of publication of application: 06.11.2013
(73) Proprietor: ABBOTT LABORATORIES, Abbott Park, IL 60064-3500 (US)
(72) Inventor: MUNDELL, Alvin Horatio, Westerville, Ohio 43082 (US); RIBBLE, Barbara Ann, Lewis Center, Ohio 43035 (US)
(74) Representative: Boult Wade Tennant
(86) International application number: PCT/US2011/064605
(87) International publication number: WO 2012/091917

(56) References cited:
- EP-A1- 2 042 855
- EP-A2- 2 031 375
- GB-A- 1 402 857
- US-A- 5 623 816
- US-A1- 2008 180 111
- EGERT S ET AL: "Spectroscopic Study of Containers and Their Content Using a High-Resolution THz System", IEEE SENSORS JOURNAL, IEEE SERVICE CENTER, NEW YORK, NY, US, vol. 10, no. 3, 1 March 2010 (2010-03-01), pages 379-383, XP011303293, ISSN: 1530-437X

## Description

### FIELD OF THE INVENTION

The present disclosure relates to non-destructive inspection of packages for contaminants. More particularly, the present disclosure relates to the non-destructive inspection of packages for spoilage by analyzing electromagnetic signals from the packages.

### BACKGROUND OF THE INVENTION

Packaged substances such as liquid products intended for human consumption are subject to spoilage (i.e., growth of undesirable bacteria or other microorganisms) in the packaging. Exemplary such liquid products include, without limitation, infant, toddler and adult nutritional formulas, fortifiers and dietary supplements. Such substances may be kept sterile from production through packaging (i.e., aseptic packaging) or may be sterilized after packaging (e.g., pasteurized). Sterilization after packaging may alter certain properties of the substance. For example, pasteurization may cause desirable proteins in a nutritional formula to break down, reducing the nutritional value of the formula.

One way of testing such packages for spoilage is to allow a relatively long incubation period before visually examining the package. Visual examination may fail to identify spoilage by certain bacteria or may fail to identify spoilage in a package having a relatively slow growing contaminant. Thus, a consumer could purchase and potentially use a package containing a spoiled substance. Further, visual examination on a commercial scale requires a substantial investment in training and man hours, and packages examined visually must be transparent and individually inspected (i.e., removed from a case containing a plurality of packages). This increases production costs and reduces flexibility in responding to changes in product demand.

Another way of inspecting packages containing a substance subject to spoiling is by destructive testing in which the package must be opened or otherwise rendered unusable such as pH analysis and culturing. Each method of destructive testing has its individual disadvantages such as not detecting early stage spoilage or not being sensitive to certain bacteria or contaminants. More importantly, however, destructive testing requires opening the package to sample the substance. The act of opening the package to sample the substance reintroduces the potential for contamination and spoilage. Thus, any test on the substance may not be valid for a given package going forward. Therefore, any packages tested are taken to be representative of a production lot and the sampled packages themselves are culled. This results in incomplete testing of a lot, excess product waste, and a significant expenditure of time which reduces flexibility in responding to changes in product demand.

In EP 2,042,855 there is described a sample inspection apparatus that includes an oscillator which generates light rays of terahertz radiation, an optical system which guides the terahertz radiation to a drug, a detection unit which detects the terahertz radiation transmitted through an object as an electrical signal, and a computer. The computer determines by analysis whether the sample contains heterogeneous or foreign substances based on the temporal waveform of the electrical signal detected by the detection unit and the predetermined temporal waveform indicating the component unique to the sample. Alternatively the computer determines the spectrum from the electrical signal detected by the detection unit, extracts plural feature values from the spectrum and determines whether the drug contains heterogeneous or foreign substances based on the extracted feature values and the feature values extracted from the predetermined spectrum due to the component unique to the drug.

In GB 1,402,857 there is described an apparatus for marking defective articles, to distinguish them from satisfactory articles in a batch, comprising a support rail mounted above a conveyor belt upon which rows or articles are being conveyed. The rail is located transversely with respect to the conveyor belt and at least one marking unit is mounted upon and adapted to be moved along the support rail. The or each unit is locatable by locating means in a position directly above a row of articles to be marked, and the or each unit is provided with marking means for applying marking to defective articles as they are conveyed past the marking units.

In US 2008/0180111 non-invasive THz spectroscopic apparatus and methods are described for detecting and/or identifying constituents such as variations in a structural entity where chemical or biological entities can reside. Position dependent scattering of THz radiation is employed to image voids and defects in the internal structure of samples, enabling the determination of contamination, spoilage or readiness of products such as wine in sealed containers.

In US 5,623,816 there is described a packaging arrangement for packaging products, such as contact lenses, in packages such as blister packs. The packaging arrangement includes a rotary index table defining on its upper surface a plurality of identical, radially-oriented support pallets, equally spaced apart around the rotary index table. Each support pallet is designed to support an array of individual package bases thereon, and is sequentially rotated to stop at angularly spaced radial positions in the rotary packaging machine. At a first radial position, the rotary packaging station receives blister package bases, each having a product deposited therein, and places the package bases in the support pallet then at the first radial position. At subsequent radial positions, the rotary packaging machine verifies the presence and alignment of each package base, deposits a fixed dosage of saline solution into each package base, optionally verifies that a fixed dosage of saline solution has been deposited in each package base, places a marked laminated cover over the package bases, heat seals the laminated cover to the package bases, verifies the proper positional placement of the laminated cover on the package bases, and finally unloads the completed blister packs from the rotary packaging station, for subsequent processing such as sterilization and secondary packaging.

### SUMMARY OF THE DISCLOSURE

According to the present invention there is provided an apparatus having the features of claim 1 and a method claim 10 for inspecting a case having a plurality of packages and determining whether a substance in each of the packages is spoiled.

The system includes an inspection system, a processor, and a labeling system. The inspection system includes an electromagnetic source, a plurality of electromagnetic scan systems, and a sampler. Each of the packages in the case has a corresponding electromagnetic scan system. The electromagnetic source generates an electromagnetic signal, and each of the plurality of electromagnetic scan systems directs the generated electromagnetic signal to a corresponding package in the case of packages. Each of the plurality of scan systems receives an attenuated electromagnetic signal from the corresponding package, and the sampler samples each of the received attenuated signals to generate a set of data points representative of an amplitude of the received attenuated signal over a predetermined period of time. The processor analyzes each set of data points by detecting a peak within the set of data points, shifting the data points with respect to time based on the detected peak, and determining whether the shifted data points correspond to a package containing a non-spoiled substance without generating an image from the set of data points. The labeling system marks any case having a package containing spoiled substance. Optionally, the apparatus includes robots for removing cases of packages from pallets for inspection and returning non-spoiled cases to a pallet after inspection. The apparatus may also include a culling system for removing packages marked by the labeling system from an inspection system. The inspection system is more suitably a quality inspection system that may itself broadly define a production line comprising an inspection system. The processor determines whether a package contains spoiled substance by comparing the shifted data points to at least one mathematical model.

In another embodiment, a mathematical model is generated for determining whether a substance in a package is spoiled. The substance is aseptically packaged in a plurality of packages, and a first group of the plurality of packages is inoculated with a first contaminant. A second group of the plurality of packages is maintained in an aseptic state. After an optional incubation period (e.g., 21 days), each of the plurality of packages is inspected. During inspection, an electromagnetic signal is directed at a package, an attenuated electromagnetic signal is received and sampled to generate a set of data points, a peak is detected within the set of data points, and the data points are shifted with respect to time to align the detected peak with a predetermined time. The set of shifted data points are analyzed to determine the mathematical model that maximizes discrimination between the sets of shifted data points corresponding to the packages of the first group and the sets of shifted data points corresponding to the packages of the second group. Optionally, after inspecting, each package of the second group of packages may be destructively tested to confirm the non-spoiled or aseptic state of the substance in the packages. In one embodiment, the mathematical model maximizing discrimination between the sets of data points of the two groups is determined by treating each data point in a set as an independent variable and performing an orthogonal partial least squares calculation. Optionally, additional variables may be added to each set of data points prior to determining the mathematical model. The additional variables are derived from the set of data points (e.g., a ratio of an amplitude of a first peak in the set of data points to an amplitude of a second peak in the set of data points).

Embodiments of the invention also provide systems and methods for determining a mathematical model for discriminating between a package containing spoiled substance and a package containing non-spoiled substance and an inspection system for inspecting for spoilage cases of packages of the substance using the mathematical model.

### FIGURES AND DRAWINGS

The foregoing objects, features and advantages of the present disclosure will become more apparent from a reading of the following description in connection with the accompanying drawings in which:
FIG. 1 is an example of a plot of magnitude versus time for an attenuated electromagnetic signal received from a package containing a substance subject to spoilage.
FIG. 2 is an example of a plot of magnitude versus time for a plurality of attenuated electromagnetic signals received from a plurality of packages.
FIG. 3 is a magnified section of the plot of FIG. 2.
FIG. 4 is the magnified section of the plot of FIG. 2 as shown in FIG. 3 after aligning the attenuated electromagnetic signals with respect to time based on a peak of each of the attenuated electromagnetic signals.
FIG. 5 is a flow chart showing a method of generating a mathematical model.
FIG. 6 is a plot of selected variables which maximize discrimination between vectors corresponding to packages containing an aseptic substance and packages containing a spoiled substance.
FIG. 7 is a perspective view of an inspection system for inspecting cases of packages containing a substance subject to spoiling.
FIG. 8 is a top view of the inspection system of FIG. 7.
FIG. 9 is a side view of the inspection system of FIGS. 7 and 8.
FIG. 10 is a perspective view of an inspection system for handling individual pouches containing a substance subject to spoiling.

### DESCRIPTION OF EMBODIMENTS

For the purposes of promoting an understanding of the principles of the present disclosure, reference will now be made to embodiments and specific language will be used to describe the same. It will nevertheless be understood that no limitation of the scope of the claims is thereby intended, such alteration and further modifications of the readings of the disclosure as illustrated herein, being contemplated as would normally occur to one skilled in the art to which the disclosure relates.

Articles "a" and "an" are used herein to refer to one or to more than one (*i.e.* at least one) of the grammatical object of the article. By way of example, "an element" means at least one element and can include more than one element.

Unless otherwise defined, all technical terms used herein have the same meaning as commonly understood by one of ordinary skill in the art to which this disclosure belongs.

The present disclosure provides systems and methods for rapid discrimination between packages containing a spoiled substance and packages containing non-spoiled substance. As used herein the term "substance" refers to a liquid product and more suitably a liquid intended for human consumption. The term "liquid product" means a product that is a flowable non-solid product including, for example but not limited to, aqueous solutions, solutions having a determinable viscosity, emulsions, colloids, pastes, gels, dispersions and other flowable non-solid products so as to exclude solid products such as bars and particulate products, such as powders.

Generally, a package containing such a substance is scanned when an electromagnetic signal from an electromagnetic source is directed at the package via an electromagnetic scan system and an attenuated electromagnetic signal is received by the electromagnetic scan system and sampled to generate a set of data points. In one embodiment, the electromagnetic scan system comprises a scan head that transmits the electromagnetic signal and receives a reflection of the electromagnetic signal which is the attenuated signal. In another embodiment, the electromagnetic scan system comprises a transmitter and corresponding receiver wherein the transmitter is located on a first side of the package, and the corresponding receiver is located on a second side of the package opposite the first side such that the attenuated signal is the electromagnetic signal after having passed through the package and substance. The data points from the scan are aligned with respect to time based on a peak detected within the set of data points and a predetermined time, and a determination of whether the package contains spoiled substance is made based on the set of aligned data points without generating an image from the set of data points. In one embodiment, the electromagnetic signal has a frequency in the range of about 0.02 terahertz to about 3.5 terahertz and a wavelength between about 0.3mm and 3mm, and the set of data points includes 4096 data points sampled over a predetermined period of time of 320 picoseconds.

The packaging containing the inspected substance may be generally any suitable packaging for containing substances that are subject to spoilage including, without limitation, glass or plastic bottles, plastic containers, bags or pouches constructed of films or other plastics, and other suitable packaging. In a more suitable embodiment, the package is substantially transparent with respect to at least one frequency or wavelength of the electromagnetic signal. As one non-limiting example, one such package may comprise a pouch formed from any suitable material including woven material, nonwoven material, films, laminates, or a combination thereof. More particularly, a suitable pouch may comprises a two layered laminate having an inner layer and an outer layer, with the inner layer formed from a co-extrusion of linear low density polyethylene (LLDPE) and ethylene vinyl alcohol (EVOH), and the outer layer formed from barrier coated polyethylene terephthalate (PET). In another embodiment, a pouch may comprise a three layered laminate having an inner layer, an outer layer, and an intermediate layer, with the inner layer formed from a co-extrusion of linear low density polyethylene (LLDPE) and ethylene vinyl alcohol (EVOH), the outer layer formed from barrier coated polyethylene terephthalate (PET), and the intermediate layer formed from aluminum oxide coated PET, or ethylene vinyl alcohol. Other suitable materials from which such a pouch may be constructed such as silicone dioxide and high density polyethylene are contemplated to be within the scope of this invention. It is also contemplated that in some embodiments a plurality of packages, each containing a substance that is subject to spoilage, are collectively packaged in an outer packaging or case (e.g., a cardboard box, plastic wrap or other suitable casing).

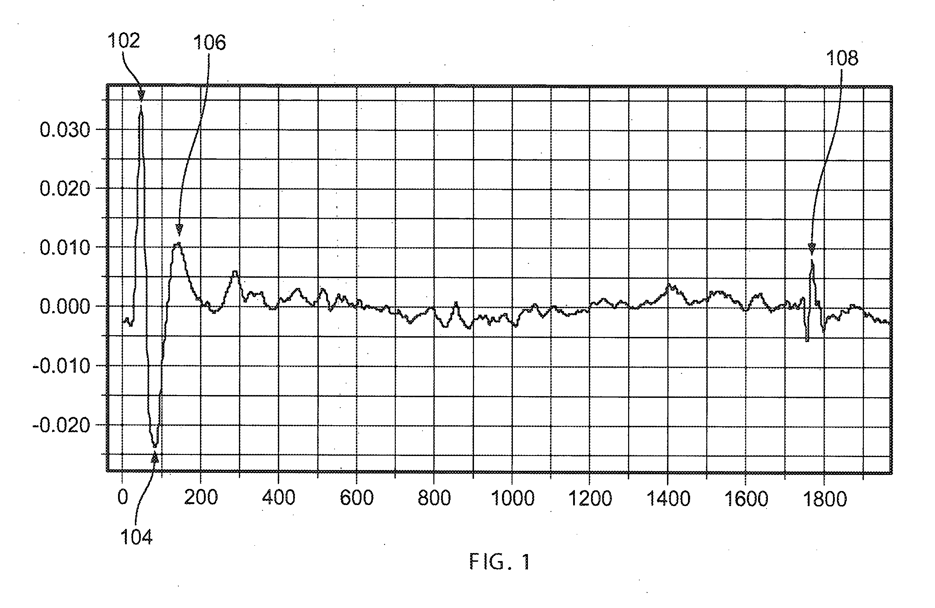

Referring to FIG. 1, one example of an electromagnetic signal attenuated by a package containing a substance is shown. In this example, the attenuated electromagnetic signal is reflected from the package containing the substance. In the plot of FIG. 1, the y-axis (i.e, the vertical axis) is magnitude, amplitude, or intensity, and the x-axis (i.e., horizontal axis) is sample number or time. A first peak 102, a second peak 104, and a third peak 106 indicate a first boundary between the package and the substance contained in the package. A fourth peak 108 indicates a second boundary between the substance and the package. The second boundary is opposite the first boundary. For example, the first boundary is at a bottom of the package while the second boundary is at a top of the package, or the first boundary is a first side of the package while the second boundary is a second side of the package opposite the first side.

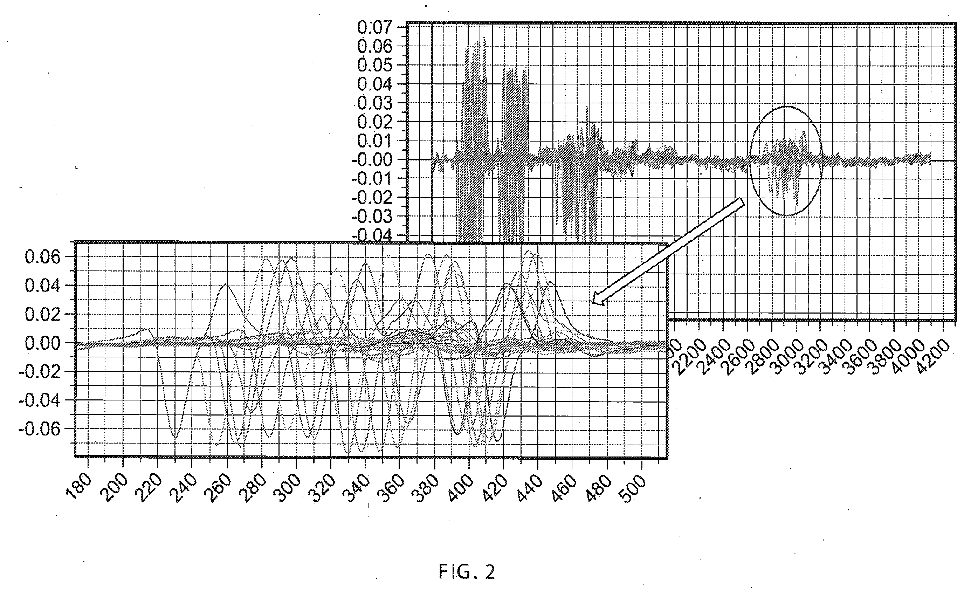

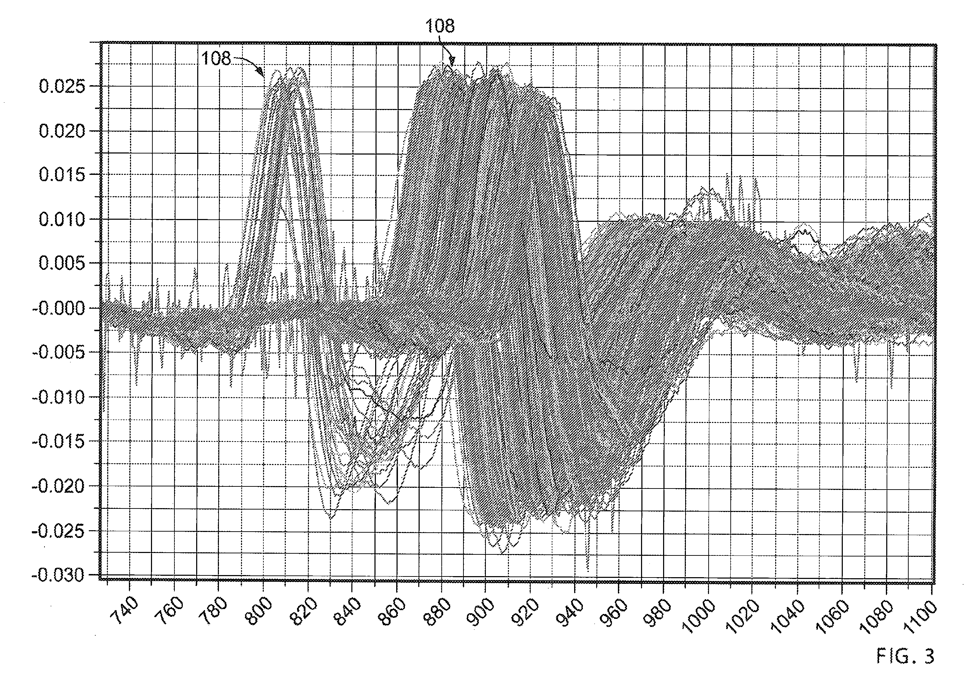

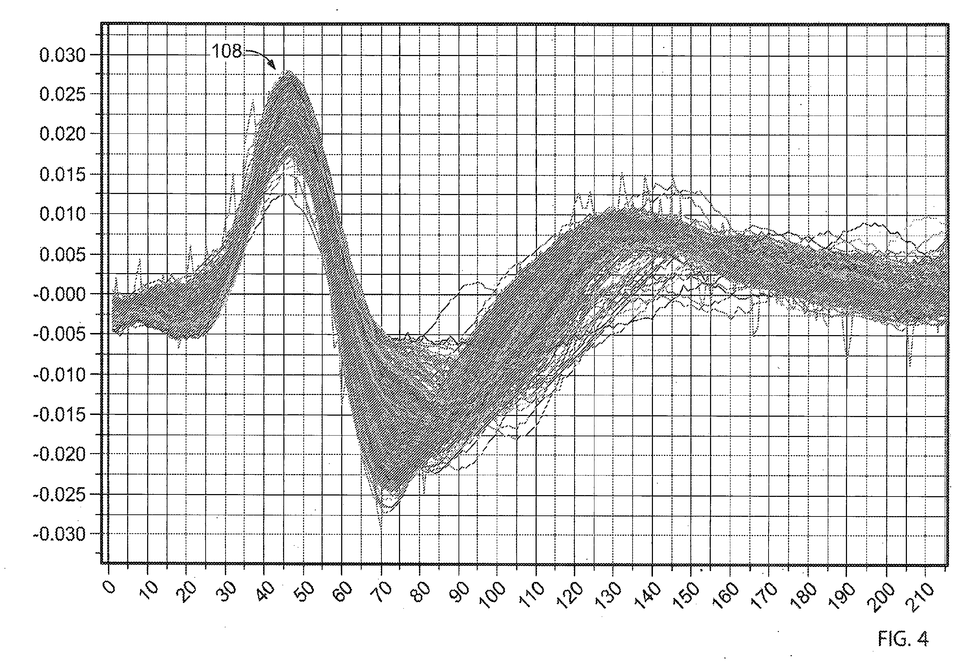

Referring to FIG. 2, sets of data points generated from scanning a plurality of packages are plotted on common axes. As in FIG. 1, the y-axis is magnitude, amplitude, or intensity, and the x-axis is sample number or time. Even though care is taken to maintain a predetermined spacing and alignment between the scan head and each package, spatial variation may still occur and presents itself in the plot as temporal variation. The fourth peak 108 of the sets of data points in FIG. 2 is shown magnified in FIG. 3. A processor uses a mathematical algorithm to identify a peak (e.g., the first peak 102 or the fourth peak 108) within each set of data points, and the temporal location of each of the data points in the data set is shifted such that the peak aligns with a predetermined time or sample number. Thus, misalignment of attenuated electromagnetic signals with respect to time as is eliminated as a variable when comparing sets of data points. FIG. 4 shows the fourth peak 108 of the sets of data points of FIG. 3 after alignment of the data points. In one embodiment, the peak for aligning or shifting the set of data points is detected by determining the data point in the set of data points having the greatest magnitude or intensity. In another embodiment, the peak is detected by determining the average for a predetermined number of adjacent data points and determining that the peak is centered at the data point in the center of the group of adjacent data points having the highest average. Other mathematical peak finding algorithms are contemplated within the scope of this disclosure. In one embodiment, MATLAB is used to detect peaks within the set of data points and shift the data points. MATLAB is available from and a registered trademark of The MathWorks, Inc.

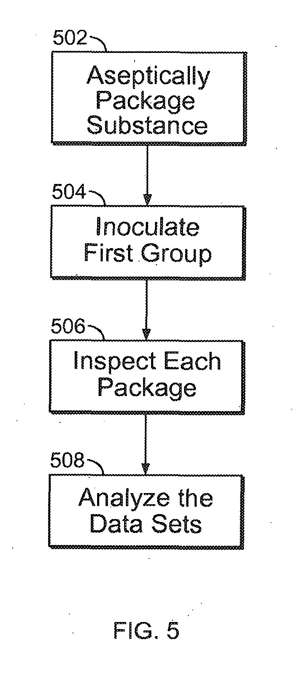

Referring to FIG. 5, a method of generating a mathematical model for determining whether a substance in a package is spoiled begins at 502. At 502, a substance subject to spoilage is aseptically packaged in a plurality of packages. It is contemplated that the packages may be sterilized by means other than aseptic packaging such as by pasteurization or other suitable sterilization technique. At 504, a first group of the plurality of packages is inoculated with a first contaminant (i.e., the substance contained therein is intentionally subjected to spoilage). In one embodiment, the first contaminant is at least one of B. Subtilis, S. Epidermidis, L. Casei, E. Cloacae, Saccharomyces Cerevisiae, and/or C. Sporogenes. A second group of the plurality of packages is maintained in an aseptic or sterile (i.e., non-inoculated) state. In one embodiment at 504, a third group of the plurality of packages is inoculated with a second contaminant. Optionally, an incubation period (e.g., 21 days) follows 504. At 506, each package of the plurality of packages is inspected or scanned (i.e., a set of data points is collected from each package). Inspecting each package includes generating an electromagnetic signal at an electromagnetic source, directing the electromagnetic signal at the package, receiving an attenuated electromagnetic signal, sampling the attenuated electromagnetic signal to generate a set of data points, detecting a peak within the set of data points, and shifting the data points based on the detected peak. The data points are shifted such that the detected peak is aligned with a predetermined time. Following inspection at 506, the second group of the plurality of packages may be tested by any means including destructive testing in order to ensure that the substance contained therein has not spoiled.

At 508, the sets of data points generated from inspecting the plurality of packages is analyzed to determine a mathematical model that maximizes discrimination between the second group and any other groups of the plurality of packages (i.e., any groups of the plurality of packages inoculated with a contaminant such that the substance contained by the package is spoiled). In one embodiment, the mathematical model is derived by treating each data point within a set of data points as an independent variable and performing a matrix or multi-dimensional (e.g., 20 dimensions) vector analysis based on all of the variables. In one embodiment, the analysis includes an orthogonal partial least squares analysis to determine the mathematical model that provides the greatest discrimination between sets of data corresponding to spoiled substance and sets of data corresponding to non-spoiled substance. SIMCA P⁺ is a software package developed by and available from Umetrics/MKS Systems which may be used to determine the relationship between variables maximizing discrimination. In one embodiment, independent variables are added to the sets of data points including an amplitude of the first peak 102, an amplitude of the second peak 104, an amplitude of the third peak 106, a ratio of the amplitude of the first peak 102 to the amplitude of the second peak 104, a ratio of the amplitude of the third peak 106 to the amplitude of the second peak 104, a ratio of the amplitude of the first peak 102 to the amplitude of the third peak 106, a position of the second peak 104, a distance between the second peak 104 and the first peak 102, a distance between the third peak 106 and the second peak 104, and a width of a peak.

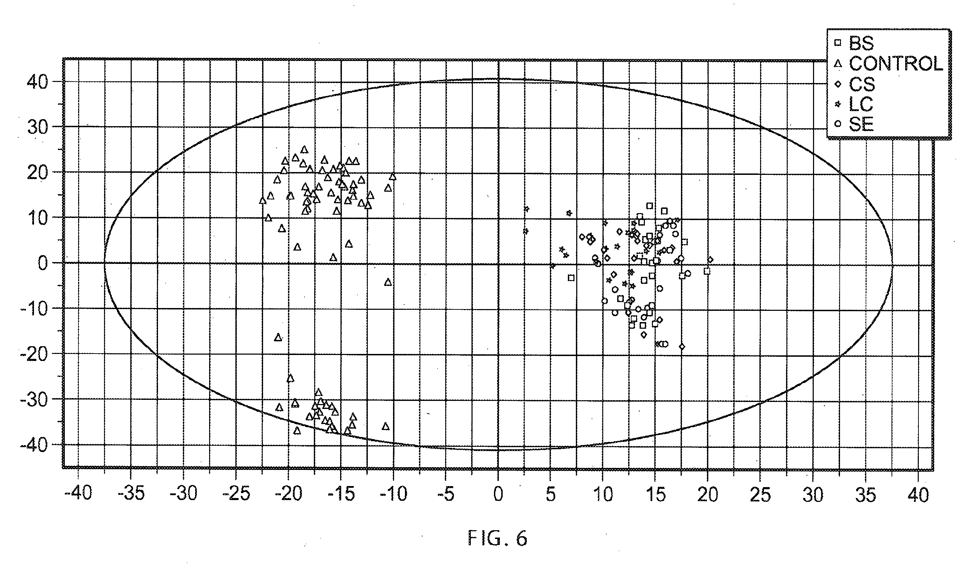

Referring now to FIG. 6, a 2 dimensional graphic representation of the multi-dimensional vectors representing the sets of data points gathered in the method of FIG. 5 shows the clustering and discrimination between packages containing spoiled substance and packages containing non-spoiled substance. The four axes shown are arbitrary due to the multi-dimensional (e.g., 20 dimensions) nature of the analyzed vectors or matrices. In FIG. 6, BS corresponds to a package inoculated with B. Subtilis, SE corresponds to a package inoculated with S. Epidermidis, LC corresponds to a package inoculated with L. Casei, CS corresponds to a package inoculated with C. Sporogenes, and CONTROL corresponds to a package maintained in an aseptic or non-spoiled state (i.e., a test control).

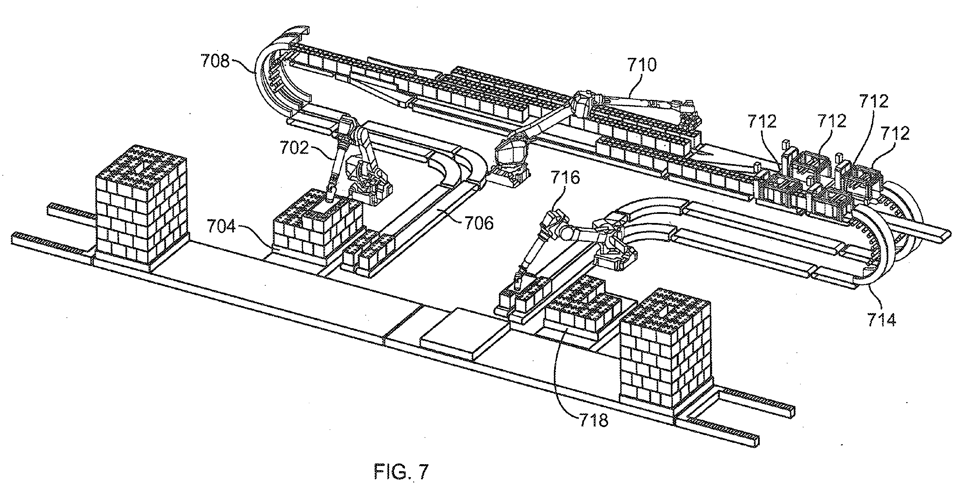

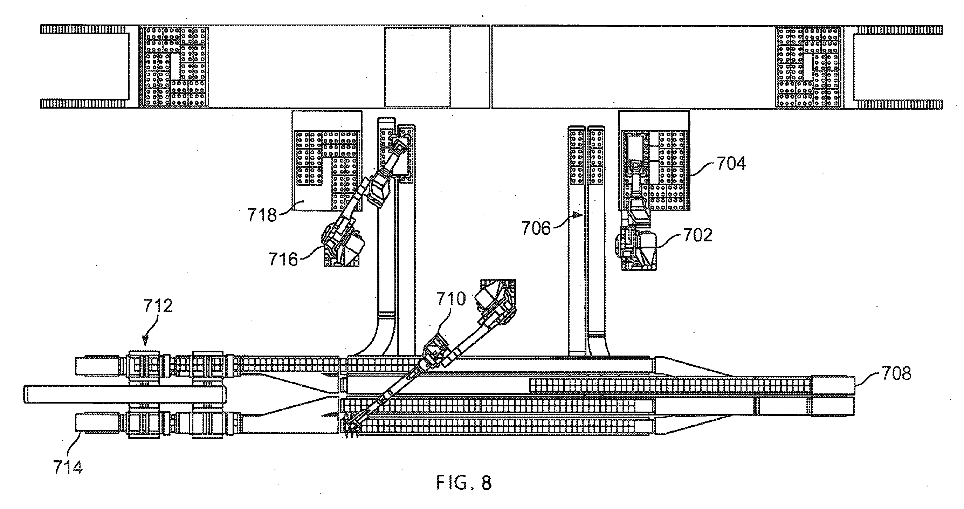

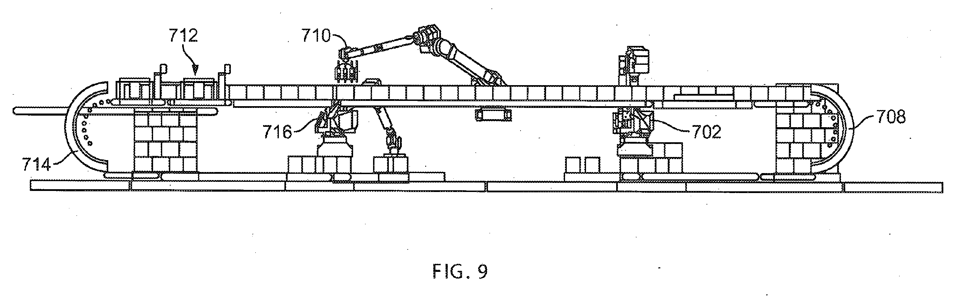

Referring now to FIGS. 7-9, various views of an inspection system for inspecting a packaging case having a plurality of packages each containing a substance subject to spoilage are shown according to one embodiment of the disclosure. Corresponding reference characters indicate corresponding parts throughout FIGS. 7-9. In one embodiment, the cases may be inspected at least 21 days after being manufactured to ensure that any contaminants and thus spoilage is detected by the apparatus. A first robot 702 removes cases of packages from a first pallet 704 and places the cases on a conveyor belt 706. The conveyor belt 706 transfers each case in turn to a first inversion system 708 for inverting each case. An inspection system robot 710 having a plurality of scan heads scans each case, and a labeling system on the inspection system robot 710 marks any cases determined to have packages containing a spoiled substance.

In one embodiment, the scan heads each receive an electromagnetic signal from one common electromagnetic source and serially scan the packages, and in another embodiment, the scan heads each have a corresponding electromagnetic source generating the electromagnetic signal. Not shown is a processor for analyzing sets of data points obtained by the inspection system robot 710 to determine whether and which packages in a case contain spoiled substance without generating an image from the sets of data points. In one embodiment, the labeling system marks a case by identifying the case in a database, and in another embodiment, the labeling system marks a case by placing ultraviolet reactive print on the case. A culling system 712 comprises redundant systems for identifying marked cases by querying the database or by exposing the cases to ultraviolet light and detecting ultraviolet reactive print on the case. The culling system 712 removes cases that it identifies as marked from the inspection system. A second inversion system 714 rights the cases that are not removed from the inspection system by the culling system 712, and a second robot 716 places the righted cases on a second pallet 718. It is contemplated that one robot may perform the function of both the first robot 702 and the second robot 716 within the scope of this disclosure.

The inspection system robot 710 has a head that includes both a plurality of scan heads, and the labeling system. In one embodiment, the number of scan heads matches the number of packages in a case. In another embodiment, the inspection system robot 710 has 8 scan heads while the cases may each contain 6 or 8 packages, and the inspection system robot 710 has a throughput of 72 cases per minute. In one embodiment, each case is allowed to settle for at least 20 seconds after inversion by the first inversion system 708 before inspection, and the inspection system robot 710 and area of the conveyor belt 706 where the cases are inspected are isolated for vibration.

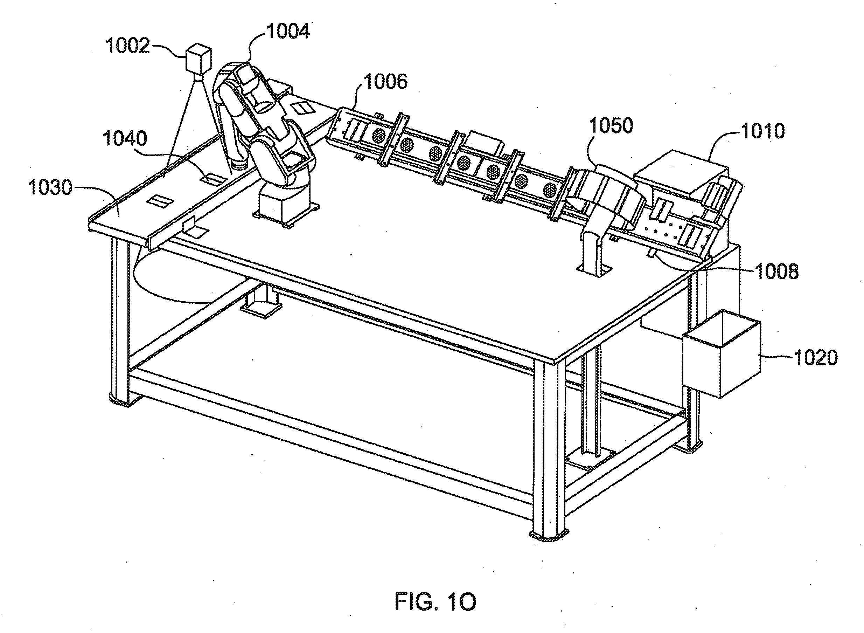

Referring to FIG. 10, one embodiment of an inspection system for inspecting pouches containing a substance subject to spoiling includes a camera 1002 and handling arm 1004. When the camera 1002 detects a pouch 1040 on a conveyor belt 1030, the handling arm 1004 is guided to the pouch 1040 by the camera 1002, and the handling arm 1004 places the pouch 1040 on a vacuum conveyor belt 1006. The pouch 1040 is passed between the vacuum conveyor belt 1006 and a scan head 1050 mounted on a rotating assembly. The rotating assembly presses against the pouch 1040 to flatten the pouch 1040 and control the distance between the scan head 1050 and the pouch in order to improve measurement precision. If the pouch 1040 is determined to be not spoiled, an air nozzle 1008 blows the pouch into a first bin 1010, and if the pouch 1040 is determined to be spoiled, the pouch 1040 is dropped into a second bin 1020.

As used herein, a processor refers to any computing device capable of executing computer executable instructions to accomplish the function of the processor described herein. Examples of a processor are a personal computer, a server, a distributed computing environment, a computing device, and an application specific integrated circuit.

From the foregoing description, it will be apparent that an improved spoilage detection system and methods have been provided. Variations and modifications of the herein described systems, apparatuses, methods and other applications will undoubtedly suggest themselves to those skilled in the art. Accordingly, the foregoing description should be taken as illustrative and not in a limiting sense.

Any patents or publications referenced in this specification are indicative of the levels of those skilled in the art to which the invention pertains.

## Claims

1. An apparatus for inspecting a case having a plurality of packages and determining whether a substance in each of the packages is spoiled, said apparatus comprising:
an inspection system (710) comprising:
an electromagnetic source for generating an electromagnetic signal;
a plurality of electromagnetic scan systems, each electromagnetic scan system corresponding to a package of the plurality of packages, said electromagnetic scan system for receiving the generated electromagnetic signal, directing the received signal at a corresponding package, and receiving an attenuated electromagnetic signal from the corresponding package;
a processor for determining whether each package contains spoiled substance as a function of the attenuated electromagnetic signal received from the package; and
a sampler for sampling the received attenuated electromagnetic signal and generating a set of data points representative of an amplitude of the
received attenuated electromagnetic signal over a predetermined period of time; and
a labeling system for marking a case having at least one package containing spoiled substance, wherein:
the processor analyzes each set of data points without generating an image from the data points, and
wherein the processor:
detects a peak (102,104,106,108) within the set of data points,
shifts the data points with respect to time based on the detected peak (102,104,106,108) in order to align the detected peak (102,104,106,108) with a predetermined time, and
compares the set of shifted data points to at least one mathematical model to determine whether the package contains spoiled substance.

2. The apparatus of claim 1 further comprising a culling system (712) for removing cases marked by the labeling system from a production line (706), said production line (706) comprising the inspection system (710), wherein marking a case comprises identifying the case in a database and wherein the culling system (712) accesses the database to identify cases to be removed from the production line.

3. The apparatus of claim 1 further comprising a culling system (712) for removing cases marked by the labeling system from a production line (706), said production line (706) comprising the inspection system (710), wherein the labeling system marks a case by placing a marking on the case in ultraviolet light reactive print, and wherein the culling system (712) comprises an ultraviolet light source and scanner for detecting the mark on the case.

4. The apparatus of claim 1 further comprising a first robot (702) for removing cases from a pallet (704) for inspection by the inspection system (710), and a second robot (716) for placing inspected cases on a pallet (718).

5. The apparatus of claim 1 wherein the labeling system marks the case to identify which of the packages in the case contain spoiled substance.

6. The apparatus of claim 1 wherein the electromagnetic signal is in the range of 0.02 terahertz to 3.5 terahertz.

7. The apparatus of claim 1 wherein:
the substance is aseptically packaged into the package;
the substance is a biological fluid; and
the at least one mathematical model corresponds to a package containing non-spoiled substance.

8. The apparatus of claim 1 wherein the package comprises at least one of linear low density polyethylene, barrier coated polyethylene terephthalate, aluminum oxide coated polyethylene terephthalate, ethylene vinyl alcohol, silicone oxide, and high density polyethylene.

9. The apparatus of claim 1 wherein the processor determines whether the substance in the package is spoiled by determining that the substance in the package comprises at least one of B. Subtilis, S. Epidermidis, L. Casei, E. Cloacae, Saccharomyces Cerevisiae, or C. Sporogenes.

10. A method for inspecting a case having a plurality of packages and determining whether a substance in each of the packages is spoiled, said method comprising:
selecting a case having a plurality of packages, wherein:
a substance is aseptically packaged into the packages,
the substance is a biological fluid, and
the packages are substantially transparent with respect to a frequency of an electromagnetic signal;
generating the electromagnetic signal;
scanning the case, said scanning comprising:
receiving the generated electromagnetic signal at one of a plurality of electromagnetic scan systems, each electromagnetic scan system corresponding to one of the plurality of packages in the case,
directing the received signal at the corresponding package, and
receiving an attenuated electromagnetic signal from the corresponding package at the corresponding electromagnetic scan system;
sampling each received attenuated electromagnetic signal;
generating a plurality of sets of data points, each set of data points representative of an amplitude of a corresponding received attenuated electromagnetic signal over a predetermined period of time;
analyzing each set of data points without generating an image from the data points, said analyzing comprising:
detecting a peak (102,104,106,108) within the generated set of data points,
shifting the data points with respect to time based on the detected peak (102,104,106,108) in order to align the detected peak with a predetermined time,
comparing the set of shifted data points to at least one mathematical model, and
determining whether the package contains spoiled substance as a function of said comparing; and
marking a case having at least one package containing spoiled substance.

## Patentansprüche

1. Gerät zur Untersuchung eines Behälters mit einer Mehrzahl von Umhüllungen und zum Bestimmen, ob eine Substanz in einer jeweiligen der Umhüllungen verdorben ist, wobei das Gerät Folgendes aufweist:
ein Untersuchungssystem (710), das Folgendes aufweist:
eine elektromagnetische Quelle zur Erzeugung eines elektromagnetischen Signals;
eine Mehrzahl von elektromagnetischen Scansystemen, wobei jedes elektromagnetische Scansystem einer Umhüllung der Mehrzahl von Umhüllungen entspricht, wobei das elektromagnetische Scansystem dem Empfang des erzeugten elektromagnetischen Signals, dem Leiten des empfangenen Signals an eine entsprechende Umhüllung und dem Empfang eines gedämpften elektromagnetischen Signals von der entsprechenden Umhüllung dient;
einen Prozessor zum Bestimmen, ob eine jeweilige Umhüllung eine verdorbene Substanz enthält, als Funktion des von der Umhüllung empfangenen gedämpften elektromagnetischen Signals; und
eine Abtastvorrichtung zum Abtasten des empfangenen gedämpften elektromagnetischen Signals und zum Erzeugen eines Satzes von Datenpunkten, die repräsentativ für eine Amplitude des empfangenen gedämpften elektromagnetischen Signals über eine vorgegebene Zeitspanne sind; und
ein Kennzeichnungssystem zum Markieren eines Behälters mit wenigstens einer Umhüllung, die eine verdorbene Substanz enthält, wobei:
der Prozessor jeden Satz von Datenpunkten analysiert, ohne ein Bild aus den Datenpunkten zu erzeugen, und
wobei der Prozessor:
eine Spitze (102, 104, 106, 108) innerhalb des Satzes von Datenpunkten erfasst,
die Datenpunkte in Bezug auf Zeit basierend auf der erfassten Spitze (102, 104, 106, 108) verschiebt, um die erfasste Spitze (102, 104, 106, 108) an einer vorgegebenen Zeit auszurichten, und
den Satz verschobener Datenpunkte mit wenigstens einem mathematischen Modell vergleicht, um zu bestimmen, ob die Umhüllung eine verdorbene Substanz enthält..

2. Gerät nach Anspruch 1, das ferner ein Aussortiersystem (712) zum Entfernen von von dem Kennzeichnungssystem markierten Behältern aus einer Fertigungsstraße (706) aufweist, wobei die Fertigungsstraße (706) das Untersuchungssystem (710) aufweist, wobei das Markieren eines Behälters ein Identifizieren des Behälters in einer Datenbank aufweist, und wobei das Aussortiersystem (712) auf die Datenbank zugreift, um Behälter zu identifizieren, die aus der Fertigungsstraße zu entfernen sind.

3. Gerät nach Anspruch 1, das ferner ein Aussortiersystem (712) zum Entfernen von von dem Kennzeichnungssystem markierten Behältern aus einer Fertigungsstraße (706) aufweist, wobei die Fertigungsstraße (706) das Untersuchungssystem (710) aufweist, wobei das Kennzeichnungssystem einen Behälter durch Platzieren einer Markierung auf dem Behälter in auf ultraviolettes Licht reagierendem Druck markiert, und wobei das Aussortiersystem (712) eine Quelle für ultraviolettes Licht und einen Scanner zur Erfassung der Markierung auf dem Behälter aufweist.

4. Gerät nach Anspruch 1, das ferner einen ersten Roboter (702) zum Entfernen von Behältern von einer Palette (704) zur Untersuchung durch das Untersuchungssystem (710) und einen zweiten Roboter (716) zur Platzierung untersuchter Behälter auf einer Palette (718) aufweist.

5. Gerät nach Anspruch 1, wobei das Kennzeichnungssystem den Behälter markiert, um zu identifizieren, welche der Umhüllungen in dem Behälter eine verdorbene Substanz enthält.

6. Gerät nach Anspruch 1, wobei das elektromagnetische Signal im Bereich von 0,02 Terahertz bis 3,5 Terahertz liegt.

7. Gerät nach Anspruch 1, wobei:
die Substanz aseptisch in der Umhüllung verpackt ist;
die Substanz eine biologische Flüssigkeit ist; und
das wenigstens eine mathematische Modell einer Umhüllung entspricht, die eine nicht-verdorbene Substanz enthält.

8. Gerät nach Anspruch 1, wobei die Umhüllung wenigstens ein lineares Polyethylen niederer Dichte, ein Sperrschicht-Polyethylenterephthalat, ein mit Aluminiumoxid beschichtetes Polyethylenterephthalat, Ethylenvinylalkohol, Silikonoxid oder ein Polyethylen hoher Dichte aufweist.

9. Gerät nach Anspruch 1, wobei der Prozessor bestimmt, ob die Substanz in der Umhüllung verdorben ist, durch Bestimmen, dass die Substanz in der Umhüllung wenigstens entweder B Subtilis, S. Epidermidis, L. Casei, E. Cloacae, Saccharomyces Cerevisiae oder C. Sporogenes aufweist.

10. Verfahren zur Untersuchung eines Behälters mit einer Mehrzahl von Umhüllungen und zum Bestimmen, ob eine Substanz in einer jeweiligen der Umhüllungen verdorben ist, wobei das Verfahren Folgendes aufweist:
Auswählen eines Behälters mit einer Mehrzahl von Umhüllungen, wobei:
eine Substanz aseptisch in den Umhüllungen verpackt ist,
die Substanz eine biologische Flüssigkeit ist, und
die Umhüllungen im Wesentlichen transparent in Bezug auf eine Frequenz eines elektromagnetischen Signals sind;
Erzeugen des elektromagnetischen Signals;
Scannen des Behälters, wobei das Scannen Folgendes aufweist:
Empfangen des erzeugten elektromagnetischen Signals an einem einer Mehrzahl von elektromagnetischen Scansystemen, wobei jedes elektromagnetische Scansystem einer der Mehrzahl von Umhüllungen in dem Behälter entspricht, Leiten des empfangenen Signals an die entsprechende Umhüllung und Empfangen eines gedämpften elektromagnetischen Signals von der entsprechenden Umhüllung an dem entsprechenden elektromagnetischen Scansystem;
Abtasten eines jeden empfangenen gedämpften elektromagnetischen Signals;
Erzeugen einer Mehrzahl von Sätzen von Datenpunkten, wobei jeder Satz von Datenpunkten repräsentativ für eine Amplitude eines entsprechenden empfangenen gedämpften elektromagnetischen Signals über eine vorgegebene Zeitspanne ist;
Analysieren eines jeden Satzes von Datenpunkten, ohne ein Bild aus den Datenpunkten zu erzeugen, wobei das Analysieren Folgendes aufweist:
Erfassen einer Spitze (102, 104, 106, 108) innerhalb des erzeugten Satzes von Datenpunkten,
Verschieben der Datenpunkte in Bezug auf Zeit basierend auf der erfassten Spitze (102, 104, 106, 108), um die erfasste Spitze an einer vorgegebenen Zeit auszurichten,
Vergleichen des Satzes verschobener Datenpunkte mit wenigstens einem mathematischen Modell, und
Bestimmen, ob die Umhüllung eine verdorbene Substanz enthält, als Funktion des Vergleichens; und
Markieren eines Behälters mit wenigstens einer Umhüllung, die eine verdorbene Substanz enthält.

## Revendications

1. Appareil pour inspecter une caisse ayant une pluralité d'emballages et pour déterminer si une substance dans chacun des emballages est détériorée, ledit appareil comprenant :
un système d'inspection (710) comprenant :
une source électromagnétique pour générer un signal électromagnétique ;
une pluralité de systèmes de balayage électromagnétique, chaque système de balayage électromagnétique correspondant à un emballage de la pluralité d'emballages, ledit système de balayage électromagnétique étant destiné à recevoir le signal électromagnétique généré, à diriger le signal reçu à un emballage correspondant, et à recevoir un signal électromagnétique atténué depuis l'emballage correspondant ;
un processeur pour déterminer si chaque emballage contient une substance détériorée en fonction du signal électromagnétique atténué reçu depuis l'emballage ; et
un échantillonneur pour échantillonner le signal électromagnétique atténué reçu et générer un ensemble de points de données représentant une amplitude du signal électromagnétique atténué reçu pendant une période prédéterminée ; et
un système d'étiquetage pour marquer une caisse ayant au moins un emballage contenant une substance détériorée, dans lequel :
le processeur analyse chaque ensemble de points de données sans générer une image à partir des points de données, et
dans lequel le processeur :
détecte un pic (102, 104, 106, 108) dans l'ensemble des points de données,
décale les points de données par rapport au temps sur la base du pic détecté (102, 104, 106, 108) afin de synchroniser le pic détecté (102, 104, 106, 108) avec un temps prédéterminé, et
compare l'ensemble de points de données décalés à au moins un modèle mathématique pour déterminer si l'emballage contient une substance détériorée.

2. Appareil de la revendication 1, comprenant en outre un système de tri (712) pour retirer des caisses marqués par le système d'étiquetage d'une chaîne de production (706), ladite chaîne de production (706) comprenant le système d'inspection (710), où le marquage d'une caisse comprend l'identification de la caisse dans une base de données et où le système de tri (712) accède à la base de données pour identifier des caisses à retirer de la chaîne de production.

3. Appareil de la revendication 1, comprenant en outre un système de tri (712) pour retirer des caisses marqués par le système d'étiquetage d'une chaîne de production (706), ladite chaîne de production (706) comprenant le système d'inspection (710), où le système d'étiquetage marque une caisse en plaçant une marque sur la caisse sous forme d'impression réagissant à la lumière ultraviolette, et où le système de tri (712) comprend une source de lumière ultraviolette et un scanneur pour détecter la marque sur la caisse.

4. Appareil de la revendication 1, comprenant en outre un premier robot (702) pour retirer des caisses d'une palette (704) pour l'inspection par le système d'inspection (710), et un deuxième robot (716) pour placer des caisses inspectés sur une palette (718).

5. Appareil de la revendication 1, dans lequel le système d'étiquetage marque la caisse pour identifier lequel des emballages dans la caisse contient une substance détériorée.

6. Appareil de la revendication 1, dans lequel le signal électromagnétique se trouve dans la plage allant de 0,02 térahertz à 3,5 térahertz.

7. Appareil de la revendication 1, dans lequel :
la substance est emballée de manière aseptique dans l'emballage ;
la substance est un fluide biologique ; et
l'au moins un modèle mathématique correspond à un emballage contenant une substance non-détériorée.

8. Appareil de la revendication 1, dans lequel l'emballage comprend au moins l'un parmi le polyéthylène linéaire basse densité, le polyéthylène téréphtalate revêtu d'une barrière, le polyéthylène téréphtalate revêtu d'oxyde d'aluminium, l'éthylène alcool vinylique, l'oxyde de silicone, et le polyéthylène haute densité.

9. Appareil de la revendication 1, dans lequel le processeur détermine si la substance dans l'emballage est détériorée en déterminant que la substance dans l'emballage comprend au moins un micro-organisme parmi B. Subtilis, S. Epidermidis, L. Casei, E. Cloacae, Saccharomyces Cerevisiae, ou C. Sporogenes.

10. Procédé pour inspecter une caisse ayant une pluralité d'emballages et pour déterminer si une substance dans chacun des emballages est détériorée, ledit procédé comprenant le fait :
de sélectionner une caisse ayant une pluralité d'emballages, dans lequel :
une substance est emballée de manière aseptique dans les emballages,
la substance est un fluide biologique, et
les emballages sont essentiellement transparents par rapport à une fréquence d'un signal électromagnétique ;
de générer le signal électromagnétique ;
de balayer la caisse, ledit balayage comprenant le fait :
de recevoir le signal électromagnétique généré au niveau de l'un d'une pluralité de systèmes de balayage électromagnétiques, chaque système de balayage électromagnétique correspondant à l'un de la pluralité d'emballages dans la caisse,
de diriger le signal reçu au niveau de l'emballage correspondant, et
de recevoir un signal électromagnétique atténué depuis l'emballage correspondant au niveau du système de balayage électromagnétique correspondant ;
d'échantillonner chaque signal électromagnétique atténué reçu ;
de générer une pluralité d'ensembles de points de données, chaque ensemble de points de données représentant une amplitude d'un signal électromagnétique atténué reçu correspondant pendant une période prédéterminée ;
d'analyser chaque ensemble de points de données sans générer une image à partir des points de données, ladite analyse comprenant le fait :
de détecter un pic (102, 104, 106, 108) dans l'ensemble généré de points de données,
de décaler les points de données par rapport au temps sur la base du pic détecté (102, 104, 106, 108), afin de synchroniser le pic détecté avec un temps prédéterminé,
de comparer l'ensemble de points de données décalés à au moins un modèle mathématique, et
de déterminer si l'emballage contient une substance détériorée en fonction de ladite comparaison ; et
de marquer une caisse ayant au moins un emballage contenant une substance détériorée.
